# EUROPÄISCHE PATENTSCHRIFT

(11) **EP 0 565 843 B1**
(45) Veröffentlichungstag und Bekanntmachung des Hinweises auf die Patenterteilung: **08.09.1999**
(21) Anmeldenummer: 93103249.4
(22) Anmeldetag: 01.03.1993
(51) Int. Cl.: G02B 6/42

(54) **Anordnung zur Bündelung und Einkopplung der von einem Halbleiterlaser erzeugten Strahlung in Lichtleitfasern**
Device for focussing and coupling semiconductor laser radiation into optical fibers
Dispositif pour focalisation et couplage du rayonnement d'un laser à semiconducteur dans des fibres optiques

(30) Priorität: 13.11.1992 DE 4238434; 16.04.1992 DE 4212832
(43) Veröffentlichungstag der Anmeldung: 20.10.1993
(73) Patentinhaber: Coherent Lübeck GmbH, 23569 Lübeck (DE)
(72) Erfinder: Lawrenz-Stolz, Jörg, W-2400 Lübeck (DE)
(74) Vertreter: Klunker . Schmitt-Nilson . Hirsch

(56) Entgegenhaltungen:
- DE-A- 3 207 988
- PATENT ABSTRACTS OF JAPAN vol. 6, no. 255 (P-162)14. Dezember 1982 & JP-A-57 150 810 ( FUJITSU ) 17. September 1982
- SPIE (OPTICAL SPACE COMMUNICATION 1989) Bd. 1131, 24. April 1989, BELLINGHAM , US Seiten 130 - 142 STREIFER ET AL.
- IEEE JOURNAL OF QUANTUM ELECTRONICS Bd. 28, Nr. 4, April 1992, NEW YORK US Seiten 952 - 965 ENDRIZ ET AL.
- PATENT ABSTRACTS OF JAPAN vol. 7, no. 269 (M-259)30. November 1983 & JP-A-58 148 777 ( KANZAKI SEISHI K.K. ) 3. September 1983
- OPTICS LETTERS Bd. 13, Nr. 4, April 1988, WASHINGTON US Seiten 306 - 308 BERGER ET AL.

## Beschreibung

Die Erfindung betrifft eine Anordnung zur Bündelung und Einkopplung der von Halbleiterlasern (Laser-Diode) erzeugten Strahlung in Lichtleitfasern, insbesondere Multimode-Lichtleitfasern, wobei im Bereich zwischen Abstrahlflächen der Laser und Lichteintrittsseiten der Lichtleitfasern ein strahlungsbündelndes Element in Form einer Zylinderlinse angeordnet ist, die im wesentlichen parallel zur Multimode-Richtung der Abstrahlflächen der Halbleiterlaser ausgerichtet ist.

Schließlich betrifft die Erfindung eine Anordnung zur Bündelung und Einkopplung der von einem Halbleiterlaser-Array (Laserdioden-Array) oder einer zweidimensionalen Halbleiterlaser-Struktur (Laserdioden-Stack) erzeugten Emissionen in eine entsprechende Anzahl von individuellen Lichtleitfasern, insbesondere Multimode-Lichtleitfasern, wobei im Bereich zwischen der Abstrahlfläche des Laserdioden-Arrays oder - Stacks und den einzelnen Lichteintrittsseiten der Lichtleitfasern ein optisches System zur Strahlungsbündelung vorgesehen ist, entsprechen dem Oberbegriff des Anspruchs 12.

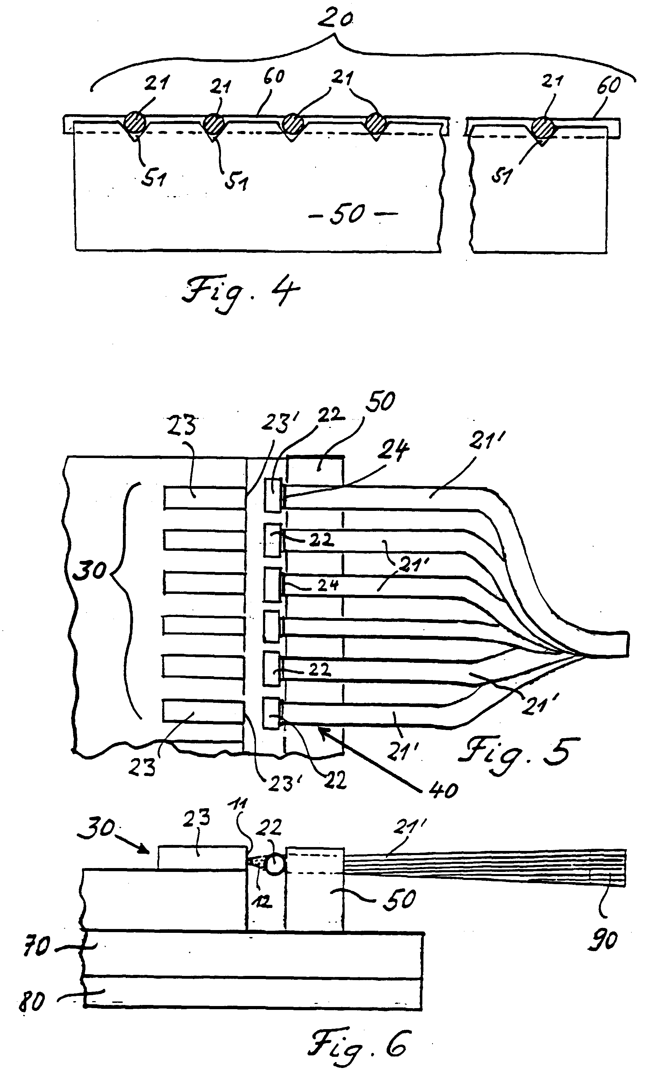

Aus der Literaturstelle "Applied Optics", Vol. 16, Nr. 7, July 1977, S. 1966-1970, ist bereits eine Anordnung mit den Merkmalen des Oberbegrifs des Patentanspruchs 1 bekannt. Gemäß dieser Literaturstelle (vgl. insbesondere die dortige Figur 5 auf S. 1968) wird für die Kopplung eines Halbleiterlasers, z.B. einer GaAs-Diode, und einer Lichtleitfaser mit rundem, bzw. kreisförmigem Querschnitt ein speziell ausgebildetes Trägerelement beschrieben, welches gleichzeitig die Zylinderlinse zur Einkopplung der von der GaAs-Diode erzeugten Laserstrahlung in die nachfolgend angeordnete Lichtleitfaser trägt. Dieses im wesentlichen zylindrische Trägerelement ist auf seiner Oberseite mit einer vorgegebenen Anzahl von V-nutenförmigen Ausnehmungen oder Aussparungen versehen, welche entsprechend der vorgesehenen gegenseitigen Kopplung zwischen Laser, Zylinderlinse und Lichtleitfaser und zur Gewährleistung der erforderlichen optischen Justierung dieser Elemente miteinander durch mechanische, d.h. abtragende Bearbeitung des Materials des Trägerelements, z.B. Kupfer, vorgebildet sind. Aufgrund dieser Gestaltung des Trägerelements lassen sich die auf die GaAs-Diode folgenden optischen Elemente, d.h. die Zylinderlinse sowie die Lichtleitfaser in diese vorgenannten Ausnehmungen einfügen, wobei die gegenseitige Justierung dieser Elemente davon abhängig ist, mit welcher Präzision die entsprechenden Ausnehmungen in dem Trägerelement herausgearbeitet worden sind. Hierbei sind natürlich nur sehr geringe Toleranzen zulässig, um letztendlich die notwendige Justierung erzielen zu können.

Somit sind die mechanischen Anforderungen an die Toleranzen eines solchen Trägerelements sowie insbesondere die Anforderung an die Positioniergenauigkeit der GaAs-Diode in bezug auf die darauffolgenden optischen Elemente außerordentlich hoch, da insbesondere diese Laserdiode auf die Oberfläche des Trägerelements aufgebracht und befestigt werden muß, beispielsweise durch Klebung oder Lötung. Hieraus folgt, daß die Herstellung und Bearbeitung eines solchen Trägerelements sehr aufwendig ist, um die erforderlichen sehr geringen Toleranzen einhalten zu können.

Diese bekannte Anordnung soll dazu dienen, möglichst viel der von einer einzigen Halbleiter-Diode emittierten Strahlung in den Kern der Lichtleitfaser, z.B. einer Multimode-Faser mit rundem Querschnitt zu fokussieren. Es ist hierbei nicht daran gedacht, ein Lasermodul mit größtmöglicher Leistungs- und Strahlqualität bereitzustellen.

Darüber hinaus ist aus "Applied Optics", Vol. 17, Nr. 3, 1978, S. 479 ff. eine Anordnung bekannt, bei dem jeder einzelne Laser eines monolithischen Laserdioden-Arrays mittels einer gemeinsamen Zylinderlinse an eine große Anzahl von individuellen Lichtleitfasern angekoppelt wird. Es wird hierbei ebenfalls ein Trägerelement in Form eines Silicium-Substrates verwendet, wobei in ähnlicher Weise wie bei der weiter oben erläuterten, bekannten Kopplungsanordnung zur Kopplung zwischen einer Laserdiode und einer Lichtleitfaser in diesem Silicium-Substrat eine vorgegebene Anzahl von V-förmigen Ausnehmungen entsprechend der beabsichtigten Anordnung von Zylinderlinse und Lichtleitfasern vorgebildet ist.

Auch in diesem Fall sind nur außerordentlich geringe Fertigungstoleranzen zulässig. Hinzu kommt, daß durch den direkten Kontakt von Zylinderlinse und Laserdioden-Array die Eigenschaften der einzelnen Laserdioden drastisch geändert werden können, d.h. es kann sehr leicht zu einer mechanischen Zerstörung der Laserdioden beim Einbringen der Zylinderlinse in die in dem Silicium-Substrat vorgesehene V-Grube kommen.

Bei der zuletzt beschriebenen Anordnung ist daher der Fertigungsaufwand ebenfalls sehr beträchtlich.

Mit Rücksicht auf den wie vorstehend geschildeten Stand der Technik liegt daher der vorliegenden Erfindung die Aufgabe zugrunde, eine verbesserte Anordnung zur Bündelung und Einkopplung der von Halbleiterlasern erzeugten Strahlung in Lichtleitfasern, insbesondere Multimode-Lichtleitfasern zu schaffen, und zwar in erster Linie mit dem Ziel, eine hocheffiziente Kopplung zwischen Multimode-Hochleistungs-Laserdioden und Multimode-Lichtleitfasern zu realisieren. Ferner liegt der Erfindung die Aufgabe zugrunde, ein Lasermodul, insbesondere ein Pumpmodul mit gegenüber bekannten Laserdioden-Modulen wesentlich größerer Leistung und mit möglichst hoher Strahlqualität zu schaffen.

Schließlich besteht noch die Forderung, die Herstellung von Anordnungen mit den Merkmalen des Oberbegriffs des Anspruchs 1 sowie entsprechende Lasermodule in einer gegenüber dem oben erläuterten Stand der Technik wesentlich vereinfachten und preisgünstigeren Art und Weise zu ermöglichen, wobei überdies alle Anforderungen an die Genauigkeit der Justierung gewährleistet werden.

Ausgehend von einer Anordnung zur Bündelung und Einkopplung der von Halbleiterlasern (Laser-Dioden) erzeugten Strahlung in Lichtleitfasern, mit den weiteren Merkmalen des Oberbegriffs des Anspruchs 1, wird die an erster Stelle genannte Aufgabe erfindungsgemäß dadurch gelöst, daß die Zylinderlinse, deren Länge im wesentlichen der Breite eines die Abstrahlfläche definierenden Strahlaustrittsfensters der Halbleiterlaser angepaßt ist und deren Durchmesser im wesentlichen in der Größenordnung des Kerndurchmessers der Lichtleitfaser liegt, vorzugsweise kleiner als dieser Kerndurchmesser ist, unmittelbar auf die Lichteintrittsseiten der im wesentlichen rechtwinklig zur Ausrichtung der Zylinderlinse verlaufenden Lichtleitfasern aufgeklebt oder mit diesen Lichteintrittsseiten verschmolzen bzw. an diese angeschmolzen ist.

Weitere vorteilhafte Ausgestaltungen dieser erfindungsgemäßen Anordnung ergeben sich aus abhängigen Ansprüchen.

Ferner kann aufgrund der vorliegenden Erfindung ein Laser-modul hoher Leistung und hoher Strahlqualität bei kleiner numerischer Apertur dadurch erhalten werden, daß eine vorgegebene Anzahl von einzelnen, nach der vorliegenden Erfindung ausgebildeten Anordnungen zu einem Modul vereinigt werden, wobei die entsprechenden einzelnen Lichtleitfasern derart gebündelt werden, daß die freien Enden, d.h. also die Lichtaustrittsseiten sämtlicher Lichtleitfasern in einer beliebig wählbaren bzw. vorgebbaren Konfiguration angeordnet sind.

Ein Lasermodul der wie im Vorangehenden charakterisierten Art läßt sich in bevorzugter Weise als Pumplaser für Systeme mit gepumpten Festkörperlasern einsetzen, wobei die an den Lichtaustrittsseiten der gebündelten Lichtleitfasern insgesamt erhaltene Laserstrahlung als Pumpenergie für den nachfolgend angeordneten, gepumpten Laser dient.

Ein derartiges optisches Pumpen (longitudinal und transversal) kann beispielsweise mit und ohne Frequenzvervielfachung durchgeführt werden, beispielsweise zum Pumpen eines Neodym-YAG-Lasers. Ferner besteht die Möglichkeit, einen Lasermodul der im Vorangehenden charakterisierten Art als Laser insbesondere für medizinische Anwendungen oder für die Materialbearbeitung zur verwenden. In solchen Fällen wird dann die an den Lichtaustrittsseiten der gebündelten Lichtleitfasern insgesamt erhaltene Laserstrahlung als Energie für den jeweiligen medizinischen Behandlungsprozeß oder den jeweiligen Materialbearbeitsvorgang genutzt.

Dabei können die Lichtaustrittsseiten der gebündelten Lichtleitfasern vorzugsweise in einer matrixförmigen Anordnung, oder aber auch in einer zeilenförmigen Anordnung zusammengefaßt werden.

Weiterhin können durch eine vorzugsweise selektive und individuelle Ansteuerung der einzelnen Diodenlaser und durch Abbildung auf einem Gegenstand beliebige Zeichen in Matrixform auf diesem Gegenstand als "Lasermarkierung" aufgebracht werden.

In entsprechender Weise können Materialbearbeitungsstellen in Matrixform rein durch die elektrische Ansteuerung der einzelnen Diodenlaser vorgewählt werden, was insbesondere bei der Mikromaterialbearbeitung, z.B. beim Löten, Schweißen oder Bohren, besondere Vorteile bringt.

Eine weitere, im Rahmen der vorliegenden Erfindung liegende Möglichkeit zur Ausführung eines Lasermoduls besteht darin, daß anstelle von einzelnen Anordnungen der weiter oben erläuterten Art mit jeweils einzelnen Laserdioden die abstrahlenden Flächen von Laserdioden-Arrays oder aber auch von zweidimensionalen Strukturen, sogenannten Laserdioden-Stacks in entsprechende Lichtleitfasern eingekoppelt und zusammengefaßt werden.

Dementsprechend ist bei einer Anordnung zur Bündelung und Einkopplung der von einem Halbleiterlaser-Array oder einer zweidimensionalen Halbleiterlaser-Struktur erzeugten Emissionen in eine entsprechende Anzahl von individuellen Lichtleitfasern vorgesehen, daß das optische System zur Strahlungsbündelung durch eine der Anzahl und Anordnung der individuellen Lichtleitfasern entsprechend dimensionierte Zylinderlinse gebildet ist, die im wesentlichen parallel zu den Multimode-Abstrahlrichtungen einer jeden Laserdiode ausgerichtetet ist, deren Länge im wesentlichen der Gesamtbreite der die Abstrahlflächen definierenden Strahlaustrittsfenster der Laserdioden angepaßt ist, und deren Durchmesser im wesentlichen in der Größenordnung des Kerndurchmessers der jeweiligen individuellen Lichtleitfaser liegt, vorzugsweise kleiner ist als dieser Kerndurchmesser, und daß diese Zylinderlinse unmittelbar auf sämtliche zugeordneten Lichteintrittsseiten der im wesentlichen senkrecht zur Ausrichtung der Zylinderlinse verlaufenden Lichtleitfasern aufgeklebt oder mit diesen Lichteintrittsseiten verschmolzen bzw. an diese angeschmolzen ist.

Weitere vorteilhafte Ausgestaltungen der Anordnung ergeben sich aus abhängigen Ansprüchen. Vorzugsweise ist im Falle des Einsatzes von Halbleiterlaser-Arrays die einzelne Zylinderlinse als eine Glasfaserlinse ausgebildet, entsprechendes gilt im Falle von zweidimensionalen Halbleiterlaser-Stacks. Der zusätzliche Vorteil derartiger Halbleiterlaser-Arrays oder -Stacks ist deren hochgenaue Fertigung und die damit verbundene geringe Lagetoleranz der abstrahlenden Flächen der entsprechenden Halbleiterlaser bzw. Laserdioden.

In bevorzugter Weise kann zwischen dem Laserdioden-Array oder -Stack und der Anordnung der Zylinderlinse des optischen Systems zur Strahlungsbündelung, in den Abstrahlrichtungen gesehen, ein relativ kleiner Luftspalt vorgesehen sein. Hierdurch wird eine gegenseitige mechanische Beeinflussung zwischen der Zylinderlinse und den entsprechend zugeordneten Laserdioden vermieden, mit dem Vorteil, daß weder die Eigenschaften dieser Laserdioden geändert werden, noch die Gefahr einer mechanischen Zerstörung der Laserdioden besteht.

Darüber hinaus besteht im Rahmen der Erfindung die sehr vorteilhafte Möglichkeit, für die Ankopplung des Laserdioden-Arrays oder -Stacks an die diesem zugeordnete Anzahl von individuellen Lichtleitfaser-Zylinderlinsen-Einheiten eine Halterung vorzusehen, welche für sämtliche dieser Einheiten gemeinsam ist. Mit Hilfe standardmäßiger Technologien (Ätztechnik, CNC-Fräsen und dgl.) lassen sich Strukturen für derartige Halterungen mit für die angestrebte Kopplung der Laserdioden-Arrays oder -Stacks an die entsprechende Anzahl von Lichtleitfasern hinreichender Toleranz im µm-Bereich in sehr einfacher und preiswerter Art und Weise herstellen.

Im Falle einer Aufklebung der Zylinderlinse beispielsweise mit Hilfe eines Epoxyklebers wird zusätzlich der besondere Vorteil erreicht, daß dieser Klebstoff an den Lichteintrittsseiten der einzelnen Lichtleitfasern die Zylinderlinse bzw. Faserlinse selbst zentriert. Ein weiterer Vorteil der unmittelbaren Aufklebemethode besteht auch darin, daß der z.B. verwendete Epoxyklebstoff sehr dünn ist und bei der von den Laserdioden beispielsweise abgegebenen Laserwellenlänge von ungefähr 810 nm eine nur sehr geringe Absorption besitzt. Überstehender Kleber trägt eher noch zur Steigerung der Koppeleffizienz bei.

Aufgrund erster Erfahrenswerte kann gesagt werden, daß typischerweise eine Koppeleffizienz von ca. 70% erreichbar ist, es ist jedoch auch eine Koppeleffizienz bis nahezu 100% theoretisch möglich.

Es versteht sich, daß auch in dem Falle, daß ein Halbleiterlaser-Array (Laserdioden-Array) oder eine zweidimensionale Halbleiterlaser-Struktur (Laserdioden-Stack) in einer Anordnung nach der vorliegenden Erfindung vorgesehen ist, diese Anordnung als Pumplaser für Systeme mit gepumpten Festkörperlasern verwendet werden kann, wobei die an den Lichtaustrittsseiten der vorzugsweise gebündelten Lichtleitfasern insgesamt zur Verfügung stehende Laserstrahlung als Pumpenergie für einen nachfolgend angeordneten, gepumpten Laser dient.

Mit ganz besonderem Vorteil können sämtliche individuellen Lichtleitfasern des entsprechenden Halbleiterlaser-Arrays oder des entsprechenden zweidimensionalen Halbleiterlaser-Stacks in der Weise gebündelt werden, daß die freien Enden, d.h. also die Lichtaustrittsseiten dieser Lichtleitfasern in beliebig wähl- bzw. vorgebbaren Konfigurationen angeordnet sind.

Hierbei kann beispielsweise eine solche Konfiguration vorgesehen sein, daß die freien Enden (Lichtaustrittsseiten) der Lichtleitfasern in einer geometrisch dichtesten Packung zusammengefaßt sind. Darüber hinaus kann eine solche Konfiguration gewählt werden, daß die freien Enden (Lichtaustrittsseiten) aller Lichtleitfasern eine im wesentlichen rechteckförmige Matrix bilden.

Andererseits besteht aber auch die Möglichkeit einer solchen Konfiguration, daß die freien Enden (Lichtaustrittsseiten) aller Lichtleitfasern eine Zeile bilden.

Schließlich kann auch noch vorgesehen sein, daß die einzelnen Laserdioden jeweils selektiv und individuell angesteuert werden, so daß die Strahlung aus einer entsprechenden Auswahl von Lichtaustrittsseiten der Lichtleitfasern austritt, wodurch beliebig wähl- bzw. vorgebbare Strahlaustrittsmuster erzeugt werden können.

Mit Hilfe einer derartigen Anordnung läßt sich beispielsweise ein Gegenstand mit einem Muster markieren, welches ganze Buchstaben, Zahlen oder sonstige Zeichen in Matrixform darstellt oder das Teile von Buchstaben, Zahlen oder sonstigen Zeichen so darstellt, daß mehrere zueinander passende Teile nebeneinandergesetzt ganze Buchstaben, Zahlen oder sonstige Zeichen in Matrixform ergeben. Hierbei können die Faserenden der zusammengefaßten Fasern entweder direkt, falls der Abstand zur Bildebene im Verhältnis zum Gesamtdurchmesser des Faserbündels relativ gering ist, oder aber mit Hilfe eines optischen Systems auf eine vorgegebene Stelle eines Gegenstands (die Bildebene) abgebildet werden, so daß auf der Oberfläche dieses Gegenstands durch das Laserlicht der leuchtenden Faserenden eine Veränderung erzeugt wird, durch welche die gewünschte Markierung des Gegenstands bewirkt wird.

Für die erfindungsgemäß ausgebildete Anordnung ergeben sich eine Reihe von bevorzugten Anwendungsmöglichkeiten, beispielsweise besteht eine solche Anwendungsmöglichkeit darin, daß die Anordnung als Pumplaser für Systeme mit gepumpten Festkörperlasern verwendet wird, wobei die an einer Lichtaustrittsseite der Lichtleitfaser erhaltene Laserstrahlung als Pumpenergie für den nachfolgend angeordneten, gepumpten Laser dient.

Die im Vorangehenden geschilderte Verwendungsmöglichkeit besteht beispielsweise auch dann, wenn eine vorgegebene Anzahl von einzelnen Anordnungen zu einem Modul vereinigt ist, wobei die entsprechenden Lichtleitfasern dieser einzelnen Anordnungen derart zusammengefaßt sind, daß die freien Enden (Lichtaustrittsseiten) aller Lichtleitfasern in einer beliebig wähl- bzw. vorgebbaren Konfiguration angeordnet sind.

Darüber hinaus läßt sich eine nach der Erfindung ausgebildete Anordnung bzw. ein entsprechender Lasermodul vorzugsweise für Gewebeabtragungen, Koagulation, Wärmebehandlung, Stimulation oder andere optische Behandlungen auf dem Gebiet der Medizin oder aber auch zur Materialbearbeitung verwenden, wobei die an der Lichtaustrittsseite der Anordnung bzw. des Lasermoduls erhaltene Laserstrahlung zur Erzeugung der vorgenannten Effekte dient.

Im Falle der Verwendung einer erfindungsgemäßen Anordnung bzw. eines erfindungsgemäßen Lasermoduls zu einer flexiblen Materialbearbeitung oder zu einer Gewebebehandlung besteht noch die Möglichkeit, daß die Faserenden der zusammengefaßten Fasern entweder direkt, falls der Abstand zur Bildebene im Verhältnis zum Gesamtdurchmesser des Faserbündels relativ gering ist, oder aber mit Hilfe eines optischen Systems auf eine vorgegebene Stelle eines Werkstücks oder eines Gewebes (d.h. in die Bildebene) abgebildet werden, an welcher sodann die erwünschte Materialbearbeitung oder Gewebebehandlung erfolgt.

Die Erfindung wird im folgenden im Rahmen von Beispielen näher erläutert, wobei auf die beigefügten Zeichnungen Bezug genommen wird.

In diesen zeigen:
Figur 1 schematisch eine Draufsicht auf eine Anordnung zur Bündelung und Einkopplung der von einer Laserdiode erzeugten Strahlung in eine Multimode-Lichtleitfaser mit kreisförmigem Querschnitt;
Figur 2 eine schematische Seitenansicht der Anordnung gemäß Figur 1;
Figur 3 eine schematische Seitenansicht einer Anordnung zur Bündelung und Einkopplung der von einer Laserdiode erzeugten Strahlung in eine Multimode-Lichtleitfaser, welche in diesem Falle einen rechteckförmigen Querschnitt besitzt;
Figur 4 schematisch eine Schnittansicht durch eine Anordnung zur Bündelung und Einkopplung der von einer Anzahl gruppenartig angeordneter Laserdioden, d.h. einem sogenannten Laserdioden-Array erzeugten Emissionen in eine entsprechende Anzahl von individuellen Lichtleitfasern mit kreisförmigem Querschnitt;
Figur 5 eine Anordnung zur Bündelung und Einkopplung der von einem Laserdioden-Array erzeugten Emissionen in eine entsprechende Anzahl von individuellen Lichtleitfasern, die in diesem Falle einen rechteckförmigen Querschnitt besitzen (in schematischer Draufsicht auf diese Anordnung);
Figur 6 eine schematische Seitenansicht der Anordnung gemäß Figur 5.

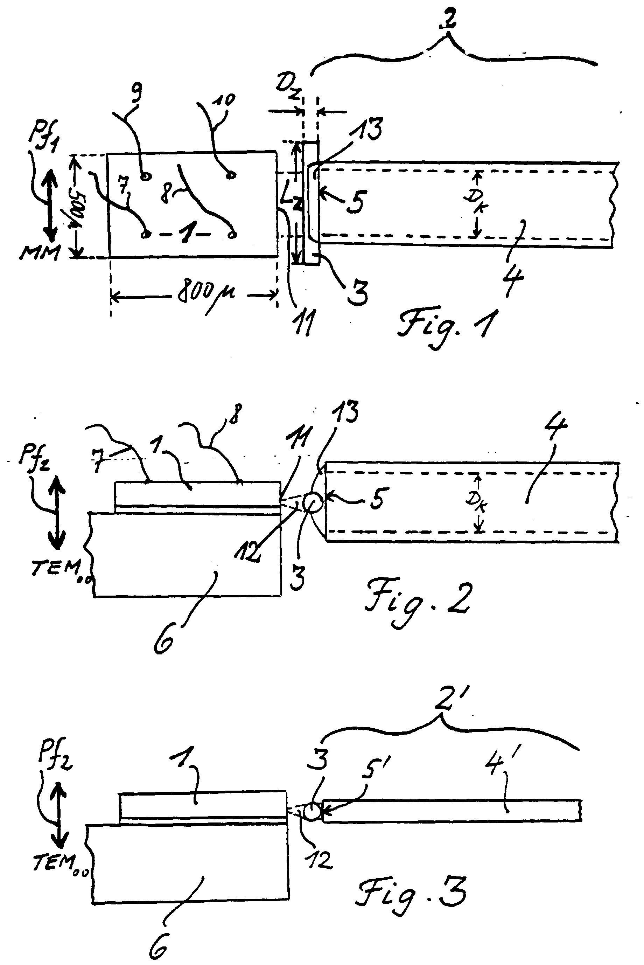

Figur 1 zeigt in Draufsicht schematisch eine Anordnung, bei der zur Bündelung und Einkopplung der von einer Laserdiode 1 in Chip-Form erzeugten Strahlung eine Multimode-Lichtleitfaser 4 vorgesehen ist, welche einen kreisförmigen Querschnitt besitzt.

Der Durchmesser des Faserkerns dieser Lichtleitfaser 4 beträgt beispielsweise 200 µm. Die Abmessungen des die Laserdiode 1 bildenen Chips betragen beispielsweise 800 µm in der Länge und 500 µm in der Breite. Es handelt sich hierbei in bevorzugter Weise um eine sogenannte Hochleistungs-Laserdiode mit einem Strahlaustrittsfenster 11, dessen Fläche beispielsweise 200 µm x 0,5 µm beträgt. Die Breite dieses Strahlaustrittsfensters 11 kann aber auch in einem Bereich zwischen 50 und 500 µm variieren, wobei die strahlende Fläche auch aus vielen kleinen, dicht gepackten, einzelnen Emittern bestehen kann. Aus dem Strahlaustrittsfenster 11 der Laserdiode 1 wird ein Multimode-Laserstrahl mit einem Öffnungswinkel von typischerweise 10 bis 12° abgegeben. Dies entspricht einer numerischen Apertur von etwa 0,1. Die Richtung dieses Multimode-Laserstrahls verläuft in der breiten Richtung der Laserdiode 1, wobei in Figur 1 durch den Pfeil Pf₁ diese Multimode-Richtung der Laserdiode 1 angedeutet ist.

In der sehr kleinen Höhe des Strahlaustrittsfensters 11 (0,5 µm) dagegen strahlt die Laserdiode 1 im Grundmode (TEM₀₀) mit einem der Höhe der leuchtenden Fläche entsprechenden Winkel von 40° bis 60° entsprechend einer numerischen Apertur von etwa 0,4.

In der Figur 1 sind ferner die elektrischen Zuleitungen zur Laserdiode 1 mit den Bezugsziffern 7, 8, 9 und 10 bezeichnet.

Die in der vorliegenden Anordnung runde Multimode-Lichtleitfaser 4 ist in der Weise angeordnet, daß ihre Lichteintrittsseite 5 dem Strahlaustrittsfenster 11 der Laserdiode 1 mit Abstand gegenuberliegt, wobei diese Lichteintrittsseite 5 im wesentlichen parallel zur Multimode-Richtung gemäß dem Pfeil Pf₁ der Abstrahlfläche der Laserdiode 1 ausgerichtet ist.

Ferner ist die Lichteintrittsseite 5 der Multimode-Lichtleitfaser 4 mit einem strahlungsbündelnden Element in Form einer Zylinderlinse 3 versehen, welche unmittelbar auf diese Lichteintrittsseite 5 mittels eines Epoxyklebers 13 (vgl. Fig. 2) aufgeklebt ist.

Die Zylinderlinse 3 ist somit ebenfalls im wesentlichen parallel zur Multimode-Richtung gemäß dem Pfeil Pf₁ der Abstrahlfläche des Lichtaustrittsfensters 11 der Laserdiode 1 ausgerichtet. Die Länge L_{Z} der Zylinderlinse 3 ist im wesentlichen der Breite des die Abstrahlfläche der Laserdiode 1 definierenden Strahlaustrittsfensters 11 angepaßt. Auf jeden Fall soll die Länge L_{Z} der Zylinderlinse 3 nicht geringer sein als die Breite des Strahlaustrittsfensters 11, in der Regel wird diese Länge L_{Z} noch etwas größer sein als die Breite des Strahlaustrittsfensters 11 in der Multimode-Richtung.

Aus Figur 2 ist eine schematische Seitenansicht der Anordnung gemäß Figur 1 ersichtlich. Die TEM₀₀ - Richtung der Laserdiode 1 ist hierbei durch einen Pfeil Pf₂ angedeutet.

Der Abstrahlwinkel der TEM₀₀-Richtung der Laser-diode 1 ist mit 12 bezeichnet, wobei, wie bereits erwähnt, die numerische Apertur z.B. 0,4 beträgt.

Der Durchmesser D_{Z} der Zylinderlinse 3 wird im allgemeinen so gewählt, daß sie in der Größenordnung des Kerndurchmessers D_{K} der Lichtleitfaser 4 liegt.

Bei der hier erläuterten Anordnung ist jedoch dieser Durchmesser D_{Z} der Zylinderlinse 3 in einem Bereich zwischen 80 und 100 µm gewählt und liegt somit unterhalb des Kerndurchmessers D_{K}=200 µm der Lichtleitfaser 4. Bei Verwendung von Lichtleitfasern mit rundem Querschnitt wird in bevorzugter Weise der Durchmesser D_{Z} der Zylinderlinse 3 kleiner sein als der Kerndurchmesser D_{K} der Lichtleitfaser 4.

Mit Hilfe der unmittelbar auf die Lichteintrittsseite 5 der Lichtleitfaser 4 aufgeklebten Zylinderlinse 3 wird es ermöglicht, praktisch das gesamte Licht der Laser-Diode 1 in den Kern der Lichtleitfaser 4 zu konzentrieren. Es handelt sich hierbei praktisch um eine Abbildung der TEM₀₀-Richtung der Laserdiode 1 mit Hilfe der optischen Eigenschaften der Zylinderlinse 3 auf die Lichteintrittsseite 5 der Multimode-Lichtleitfaser 4.

Vorzugsweise sind Größe und numerische Apertur der Lichtleitfaser 4 so gewählt, daß sie der Breite und dem Abstrahlwinkel bzw. der numerischen Apertur in der Multimode-Richtung der Laserdiode 1 entsprechen.

Der für die Verklebung der Zylinderlinse 3 mit der Lichteintrittsseite 5 der Lichtleitfaser 4 beispielsweise verwendete Epoxykleber, vgl. Klebeschicht 13 gemäß Figur 2, ist sehr dünn und hat eine verhältnismäßig geringe Absorption bei der hier erzeugten Laserwellenlänge von z.B. 810 nm.

Wie sich in der Praxis gezeigt hat, trägt überstehender Klebstoff eher noch zur Steigerung der Koppel-effizienz zwischen Laserdiode 1 und Lichtleitfaser 4 bei. Die erzielte Koppel-Effizienz beträgt beispielsweise 70%.

In bevorzugter Weise läßt sich für die Zylinderlinse 3 eine Faserlinse verwenden, welche im einfachsten Falle aus einem Stück üblicher Glasfaser besteht. Außerdem sind aber bereits speziell für die Abbildung von Laserdioden verwendbare Faserlinsen auf dem Markt, welche sich im Falle der vorliegenden Erfindung ebenfalls gut als Zylinderlinsen eignen.

Anstelle des Aufklebens der Zylinderlinse 3, beispielsweise einer Faserlinse, unmittelbar auf die Lichteintrittsseite 5 der Lichtleitfaser 4 kommt aber auch ein direktes Anschmelzen der Zylinderlinse 3 an die Lichtleitfaser 4 in Frage, wobei ein derartiges Anschmelzen beispielsweise mit Hilfe eines CO₂-Lasers oder eines Lichtbogens oder dgl. vorgenommen werden kann.

Ein solches Anschmelzen kommt insbesondere dann in Betracht, wenn es sich bei der Multimode-Lichtleitfaser um eine Faser mit einem im wesentlichen rechteckförmigen Querschnitt handelt, wie dies näher anhand der Figur 3 erläutert ist. Figur 3 zeigt eine der Figur 2 entsprechende Seitenansicht einer Anordnung zur Bündelung und Einkopplung der von einer Laserdiode 1 erzeugten Strahlung in eine Multimode-Lichtleitfaser 4', welche im Querschnitt rechteckig ist und die Abmessungen von beispielsweise 200 µm in der Breite und 20 µm in der Höhe besitzt. Auf die Lichteintrittsseite 5' dieser Multimode-Lichtleitfaser 4' ist wiederum unmittelbar eine Zylinderlinse bzw. Faserlinse 3 aufgeklebt, wobei in diesem Falle die Zylinderlinse 3 einen Durchmesser von beispielsweise 20 µm besitzt, entsprechend der Höhenabmessung der rechteckförmigen Lichtleitfaser 4'.

Es sind aber auch Durchmesser der Zylinderlinse 3 in der Größenordnung von 5 bis 10 µm denkbar, d.h. also in der Größenordnung des Durchmessers einer Monomode-Lichtleitfaser.

Wie aus den Figuren 2 und 3 ferner ersichtlich ist, ist die Laserdiode 1, z.B. GaAs-Diode, auf einem Trägerelement 6, z.B. einem Silicium-Substrat angeordnet.

Wie anhand der Figuren 1 bis 3 bereits im einzelnen erläutert, besteht bei der erfindungsgemäßen Anordnung das Prinzip der Kopplung in einer direkten Verklebung bzw. Verschmelzung einer Zylinderlinse mit der Strahleintrittsseite einer Lichtleitfaser, welche im wesentlichen rechtwinklig zur Ausrichtung dieser Zylinderlinse verläuft. Hierdurch wird praktisch ein fester Verbund zwischen Zylinderlinse bzw. Faserlinse und Lichtleitfaser erhalten, wobei gleichzeitig eine Vorjustierung erzielt wird, indem sich die Zylinder- bzw. Faserlinsen auf den Lichtleitfasern quasi selbsttätig zentrieren.

Infolge dessen lassen sich beispielsweise vorgefertigte, aus Zylinderlinse und Lichtleitfaser bestehende Einheiten gleichsam auf Lager halten, wobei dann bei der praktischen Anwendung in Verbindung mit einer Laserdiode nur noch eine Zentrierung dieser Einheit in bezug auf diese Diode erforderlich ist, sonst aber keine weiteren Justiermaßnahmen bezüglich dieser Kopplungseinheiten selbst.

In der Figur 1 ist eine solche, aus einer im Querschnitt kreisförmigen Lichtleitfaser 4 und einer stirnseitig aufgeklebten Zylinderlinse 3 bestehende, optische Kopplungseinheit mit der Bezugsziffer 2 bezeichnet, während in der Figur 3 entsprechend eine aus einer Lichtleitfaser 4' mit rechteckförmigem Querschnitt und einer stirnseitig aufgeklebten Zylinderlinse 3 bestehende optische Kopplungseinheit mit der Bezugsziffer 2' bezeichnet ist.

Darüber hinaus ist aus den Figuren 1 bis 3 noch ersichtlich, daß jeweils zwischen dem Strahlaustrittsfenster 11 der Laserdiode 1 und der Zylinderlinse 3 in den jeweiligen Abstrahlrichtungen gesehen, ein verhältnismäßig kleiner Luftspalt vorgesehen ist, wodurch eine Beeinträchtigung der Eigenschaften der Laserdiode bzw. deren mechanische Beschädigung oder gar Zerstörung von vornherein vermieden werden kann.

Bei der Anordnung gemäß Figur 3 kann anstelle einer Lichtleitfaser mit rechteckförmigem Querschnitt aber auch eine im wesentlichen flach ausgebildete, abgerundete Lichtleitfaser verwendet werden.

Aus den Figuren 5 und 6 ist ein Laser-Modul ersichtlich, bei dem eine Gruppe aus mehreren, nebeneinander angeordneten MultimodeHochleistungs-Laserdioden 23, d.h. also praktisch ein Laserdioden-Array 30 vorgesehen ist, wobei die von den einzelnen Laserdioden 23 erzeugten Emissionen in eine entsprechende Anzahl von individuellen Multimode-lichtleitfasern mit rechteckförmigem Querschnitt eingekoppelt werden.

Hierbei ist in analoger Weise zu den Anordnungen gemäß den Figuren 1, 2 und 3 im Bereich zwischen der Abstrahlfläche des Laserdioden-Arrays 30 und den einzelnen Lichteintrittsseiten 24 der jeweils zugeordneten Lichtleitfaser 21 mit rechteckförmigem Querschnitt ein optisches System zur Strahlungsbündelung angeordnet, welches in der vorliegenden Anordnung durch eine der Anzahl und Anordnung der individuellen Lichtleitfasern 21' entsprechende Anzahl von Anordnungen von individuellen Zylinderlinsen 22 gebildet ist.

Alle diese Zylinderlinsen 22 sind im wesentlichen parallel zu der Multimode-Abstrahlrichtung einer jeden Laser-diode 23 des Laserdioden-Arrays 30 ausgerichtet, ferner ist die Länge L_{Z} einer jeden Zylinderlinse 22 im wesentlichen der Breite eines die Abstrahlfläche definierenden Strahlaustrittsfensters 23' der jeweiligen Laserdiode 23 angepaßt, während der Durchmesser D_{Z} der Zylinderlinsen 22 im wesentlichen gleich der Abmessung in der Höhe einer jeden einzelnen der im Querschnitt rechteckförmigen Lichtleitfasern 21' ist.

Jede einzelne Zylinderlinse 22 ist jeweils unmittelbar auf die zugeordnete Lichteintrittsseite 24 der Lichtleitfasern 21' aufgeklebt, in entsprechender Weise, wie dies bereits weiter oben anhand der Figuren 1 bis 3 im Detail erläutert ist.

Es entstehen somit wiederum jeweils aus Zylinderlinse 22 und Lichtleitfaser 21' bestehende optische Koppeleinheiten, welche gemäß Figur 5 mit der Bezugsziffer 40 bezeichnet sind.

Darüber hinaus zeigen die Figuren 5 und 6, daß für die Ankopplung des Laserdioden-Arrays 30 an die diesem zugeordnete Anzahl von individuellen, aus Zylinderlinse und Lichtleitfaser bestehenden optischen Koppeleinheiten 40 eine Halterung 50 vorgesehen ist, durch welche jede einzelne der zunächst parallel nebeneinander angeordneten Lichtleitfasern 21' gehaltert wird.

Im Zuge des Zusammenbaus einer solchen Anordnung werden sodann der Laserdioden-Array 30 und die Halterung 50 zueinander justiert und sodann auf einem gemeinsamem Träger 70 befestigt, beispielsweise durch Verschraubung oder Verklebung, vgl. Figur 6.

Dieser Träger 70 ist schließlich noch auf einem Peltier-Element 80 angeordnet. Zur Einstellung der erwünschten Laserwellenlänge wird der gemeinsame Träger temperiert, wobei eine derartige Temperierung zusätzlich noch zur Stabilisierung der Kopplung beiträgt. Auch im Falle des Ausführungsbeispiels gemäß den Figuren 5 und 6 sind zwischen dem Laserdioden-Array 30 und der Anordnung der individuellen Zylinderlinsen 22 in den Abstrahlrichtungen gesehen, jeweils relativ kleine Luftspalte vorgesehen.

Von besonderer Bedeutung der aus den Figuren 5 und 6 ersichtlichen Anordnung ist nun, daß sämtliche Lichtleitfasern 21' in der Weise gebündelt werden können, daß ihre freien Enden, d.h. also ihre Lichtaustrittsseiten in einer gleichsam beliebig wähl- bzw. vorgebbaren Konfiguration angeordnet und hierbei insbesondere in einer geometrisch dichtesten Packung zusammengefaßt sind.

Dies bedeutet praktisch, daß die einzelnen Laserdioden 23 in dem Laserdioden-Array 30 zu einem Laser-Modul sehr hoher Leistung gekoppelt werden können,indem dieses Laserdioden-Array 30 zunächst an die im Querschnitt rechteckförmigen Lichtleitfasern 21' gekoppelt wird, welche sodann lichtaustrittsseitig zu einem vorzugsweise quadratischen Bündel 90 aufeinandergestapelt werden, beispielsweise zu einem Bündel mit den Kantenlängen 220 µm x 220 µm und einer numerischen Apertur von 0,11.

Aus der Figur 4 ist ein erfindungsgemäßer Ausführungsbeispiel für eine Anordnung zur Bündelung und Einkopplung der von einem Halbleiterlaser-Array erzeugten Emissionen in eine entsprechende Anzahl von individuellen Multimode-Lichtleitfasern 21 ersichtlich, wobei in diesem Falle im Bereich zwischen der Abstrahlfläche des (in Figur 4 hinter der Papierebene liegenden) Laserdioden-Arrays und den einzelnen Lichteintrittsseiten der Lichtleitfasern 21 eine einzige, durchgehende Zylinderlinse 60 vorgesehen.

Diese Zylinderlinse 60 ist beispielsweise durch ein entsprechend langes Stück einer Faserlinse gebildet, welche in der Weise dimensioniert ist, daß ihre Länge im wesentlichen der Summe der Breiten der jeweiligen, die Abstrahlflächen definierenden Strahlaustrittsfenster sämtlicher Laserdioden des Arrays angepaßt ist, wobei der Durchmesser dieser Zylinderlinse 60, in analoger Weise zu dem Ausführungsbeispiel gemäß Figur 1, vorzugsweise geringer ist als der jeweilige Kerndurchmesser einer jeden Lichtleitfaser 21.

Diese gemeinsame Zylinderlinse 60 ist wiederum auf sämtliche Lichteintrittsseiten der im wesentlichen senkrecht zur Ausrichtung der Zylinderlinse 60 verlaufenden Lichtleitfasern 21 aufgeklebt oder aber mit diesen Lichteintrittsseiten verschmolzen bzw. an diese angeschmolzen.

Die parallel nebeneinander liegenden Lichtleitfasern 21 sind wiederum durch eine Halterung 50 gehaltert, welche entsprechende V-Nuten 51 an ihrer Oberseite zum Einfügen der Lichtleitfasern 21 aufweist. Diese Halterung 50 wird sodann, in entsprechender Weise, wie dies bereits anhand der Figur 6 erläutert ist, zu dem zugeordneten Laserdioden-Array justiert, so daß anschließend wiederum diese justierte Anordnung auf einem gemeinsamen Träger befestigt werden kann, wie in Figur 6 gezeigt.

Bei dem Ausführungsbeispiel der Anordnung nach Figur 4 lassen sich sodann die Lichtaustrittsseiten der im Querschnitt runden Lichtleitfasern 21 zu einem Bündel zusammenfassen, beispielsweise werden 12 freie Lichtleitfaserenden zu einem Bündel mit einem Durchmesser von ca. 900 µm und mit einer numerischen Apertur von 0,11 und einer Ausgangsleistung von ca. 7W.

Aufgrund der beispielsweise in den Figuren 4, 5 und 6 dargestellten Anordnungen lassen sich Lasermodule hoher Leistung (bis 50 W) herstellen, welche sich sehr gut als Pumplaser für endgepumpte Festkörperlaser eignen, beispielsweise zur Anwendung in der Medizin, oder zum Löten, oder dgl. mehr.

## Patentansprüche

1. Anordnung zur Bündelung und Einkopplung der von einem Halbleiterlaser-Array, im weiteren auch Laserdioden-Array genannt, oder einer zweidimensionalen Halbleiterlaser-Struktur, im weiteren auch Laserdioden-Stack genannt, erzeugten Emissionen in eine entsprechende Anzahl von individuellen Lichtleitfasern, insbesondere Multimode-Lichtleitfasern, wobei im Bereich zwischen der Abstrahlfläche des Laserdioden-Arrays oder -Stacks und den einzelnen Lichteintrittsseiten der Lichtleitfasern ein optisches System zur Strahlungsbündelung vorgesehen ist, wobei das optische System (20) durch eine der Anzahl und Anordnung der individuellen Lichtleitfasern (21) entsprechend dimensionierte Zylinderlinse (60) gebildet ist, die im wesentlichen parallel zu den Multimode-Abstrahlrichtungen einer jeden Laserdiode (23) des Arrays (30) bzw. des Stacks ausgerichtet, deren Länge (L_{Z}) im wesentlichen der Gesamtbreite der die Abstrahlflächen definierenden Strahlaustrittsfenster der Laser-Dioden (23) angepaßt ist und deren Durchmesser (D_{Z}) im wesentlichen in der Größenordnung des Kerndurchmessers (D_{K}) der jeweiligen individuellen Lichtleitfaser (4) liegt, vorzugsweise kleiner ist als dieser Kerndurchmesser (D_{K}), **dadurch gekennzeichnet,** daß diese Zylinderlinse (60) unmittelbar auf sämtliche zugeordneten Lichteintrittsseiten (24) der im wesentlichen senkrecht zur Ausrichtung der Zylinderlinse (60) verlaufenden Lichtleitfasern (21) aufgeklebt oder mit diesen Lichteintrittsseiten verschmolzen bzw. an diese angeschmolzen ist.

2. Anordnung nach Anspruch 1, **dadurch gekennzeichnet,** daß die Zylinderlinse (60) als eine Glasfaserlinse ausgebildet ist.

3. Anordnung nach einem der Ansprüche 1 oder 2, **dadurch gekennzeichnet**, daß die Lichtleitfasern (21) jeweils einen im wesentlichen runden bzw. kreisförmigen Querschnitt besitzen.

4. Anordnung nach einem der Ansprüche 1 oder 2, **dadurch gekennzeichnet**, daß die Leitleitfasern (21) jeweils einen im wesentlichen rechteckförmigen Querschnitt besitzen.

5. Anordnung nach einem der Ansprüche 1 bis 4, **dadurch gekennzeichnet,** daß zwischen dem Laserdioden-Array (30) oder -Stack und der Zylinderlinse (60) des optischen Systems zur Strahlungsbündelung, in den Abstrahlrichtungen gesehen, ein relativ kleiner Luftspalt vorgesehen ist.

6. Anordnung nach einem der Ansprüche 1 bis 5, **dadurch gekennzeichnet**, daß für die Ankopplung des Laserdioden-Arrays (30) oder Laserdioden-Stacks an die, diesem zugeordnete Anzahl von individuellen Lichtleitfasern im Verbund mit der Zylinderlinse (60) eine für sämtliche Lichtleitfasern gemeinsame Halterung (50) vorgesehen ist und daß der Laserdioden-Array (30) bzw. der Laserdioden-Stack sowie die Halterung (50) zueinander justiert und auf einem gemeinsamen Träger (70) befestigt sind.

7. Anordnung nach Anspruch 6, **dadurch gekennzeichnet**, daß der gemeinsame Träger (70) zur Einstellung der erwünschten Laserwellenlänge temperiert wird.

8. Anordnung nach einem der Ansprüche 1 bis 7, **dadurch gekennzeichnet,** daß sämtliche individuellen Lichtleitfasern (21 bzw. 21') in der Weise gebündelt sind, daß die freien Enden, d.h. die Lichtaustrittsseiten, der Lichtleitfasern in beliebig wähl- bzw. vorgebbaren Konfigurationen angeordnet sind.

9. Anordnung nach Anspruch 8, **gekennzeichnet durch** eine derartige Konfiguration, daß die freien Enden der Lichtleitfasern (21 bzw. 21') in einer geometrisch dichtesten Packung zusammengefaßt sind.

10. Anordnung nach den Ansprüchen 8 oder 9, **dadurch gekennzeichnet,** daß die Konfiguration so gewählt ist, daß die freien Enden aller Lichtleitfasern (21 bzw. 21') eine im wesentlichen rechteckförmige Matrix bilden.

11. Anordnung nach den Ansprüchen 8 oder 9, **dadurch gekennzeichnet**, daß die Konfiguration so gewählt ist, daß die freien Enden aller Lichtleitfasern (21 bzw. 21') eine Zeile bilden.

12. Anordnung nach einem der Ansprüche 8 bis 11, **dadurch gekennzeichnet**, daß die einzelnen Laser-Dioden (23) jeweils selektiv und individuell angesteuert werden, so daß die Strahlung aus einer entsprechenden Auswahl von Lichtaustrittsseiten der Lichtleitfasern (21 bzw. 21') austritt, wodurch beliebig wähl- bzw. vorgebbare Strahlaustrittsmuster erzeugt werden können.

13. Verwendung einer Anordnung nach einem der Ansprüche 1 bis 8, als Pumplaser für Systeme mit gepumpten Festkörperlasern, wobei die an den Lichtaustrittsseiten der Lichtleitfasern insgesamt erhaltene Laserstrahlung als Pumpenergie für den nachfolgend angeordneten, gepumpten Laser dient.

14. Verwendung einer Anordnung nach einem der Ansprüche 1 bis 8 für Gewebeabtragung, Koagulation, Wärmebehandlung, Stimulation oder andere optische Behandlungen in der Medizin oder zur Materialbearbeitung, wobei die an der Lichtaustrittsseite erhaltene Laserstrahlung zur Erzeugung der genannten Effekte verwendet wird.

15. Verwendung einer Anordnung nach Anspruch 12 zur Markierung eines Gegenstandes mit einem Muster, das ganze Buchstaben, Zahlen oder sonstige Zeichen in Matrixform darstellt oder das Teile von Buchstaben, Zahlen oder sonstigen Zeichen so darstellt, daß mehrere zueinander passende Teile nebeneinandergesetzt ganze Buchstaben, Zahlen oder sonstige Zeichen in Matrixform ergeben, wobei die Faserenden der zusammengefaßten Fasern entweder direkt, wenn der Abstand zur Bildebene im Verhältnis zum Gesamtdurchmesser des Faserbündels relativ gering ist, oder mit Hilfe eines optischen Systems auf eine vorgegebene Stelle eines Gegenstandes (Bildebene) abgebildet werden, so daß auf dessen Oberfläche durch das Laserlicht der leuchtenden Faserenden eine eine gewünschte Markierung des Gegenstandes bewirkende Veränderung hervorgerufen wird.

16. Verwendung einer Anordnung nach Anspruch 12 zur flexiblen Materialbearbeitung oder Gewebebehandlung, wobei die Faserenden der zusammengefaßten Fasern entweder direkt, wenn der Abstand zur Bildebene im Verhältnis zum Gesamtdurchmesser des Faserbündels gering ist, oder mit Hilfe eines optischen Systems auf eine vorgegebene Stelle des Werkstückes oder des Gewebes (Bildebene) abgebildet werden, an welcher sodann die erwünschte Materialbearbeitung oder Gewebebehandlung erfolgt.

## Claims

1. An assembly for focusing and coupling the emissions produced by a semiconductor laser array, also referred to hereinafter as a laser diode array, or a two-dimensional semiconductor laser structure, also referred to hereinafter as a laser diode stack, into a corresponding number of individual optical fibers, in particular multimode optical fibers, with an optical system for focusing radiation being provided in the area between the radiating surface of the laser diode array or stack and the individual light entrance sides of the optical fibers, said optical system (20) being formed by a cylinder lens (60) dimensioned in accordance with the number and arrangement of the individual optical fibers (21) and being oriented substantially parallel to the multimode direction of radiation of each laser diode (23) of the array (30) or stack, the length (L_{Z}) of said cylinder lens being adapted substantially to the total width of the beam exit windows defining the radiating surfaces of the laser diodes (23), and the diameter (D_{Z}) of said cylinder lens being substantially in the order of magnitude of the core diameter (D_{K}) of the particular individual optical fiber (4), preferably smaller than said core diameter (DK), characterized in that said cylinder lens (60) is directly glued to, fused with or melted onto all associated light entrance sides (24) of the optical fibers (21) extending substantially at right angles to the orientation of the cylinder lens (60).

2. The assembly of claim 1, characterized in that the cylinder lens (60) is designed as a glass fiber lens.

3. The assembly of either of claims 1 and 2, characterized in that the optical fibers (21) each have a substantially round or circular cross section.

4. The assembly of either of claims 1 and 2, characterized in that the optical fibers (21) each have a substantially rectangular cross section.

5. The assembly of any of claims 1 to 4, characterized in that a relatively small air gap is provided between the laser diode array (30) or stack and the cylinder lens (60) of the optical system for focusing radiation, regarded in the directions of radiation.

6. The assembly of any of claims 1 to 5, characterized in that a holding means (50) common to all optical fibers is provided for coupling the laser diode array (30) or laser diode stack to the associated number of individual optical fibers in the compound with the cylinder lens (60), and the laser diode array (30) or laser diode stack and the holding means (50) are adjusted to each other and fastened to a common carrier (70).

7. The assembly of claim 6, characterized in that the common carrier (70) is tempered to adjust the desired laser wavelength.

8. The assembly of any of claims 1 to 7, characterized in that all individual optical fibers (21 or 21') are bunched so that the free ends, i.e. light exit sides, of the optical fibers are disposed in configurations that can be selected or predetermined at will.

9. The assembly of claim 8, characterized by a configuration such that the free ends of the optical fibers (21 or 21') are combined in a geometrically closest packing.

10. The assembly of claim 8 or 9, characterized in that the configuration is selected such that the free ends of all optical fibers (21 or 21') form a substantially rectangular matrix.

11. The assembly of claim 8 or 9, characterized in that the configuration is selected such that the free ends of all optical fibers (21 or 21') form a line.

12. The assembly of any of claims 8 to 11, characterized in that the individual laser diodes (23) are driven selectively and individually so that the radiation emerges from a corresponding selection of light exit sides of the optical fibers (21 or 21'), allowing the production of beam exit patterns that can be selected or predetermined at will.

13. Use of the assembly of any of claims 1 to 8 as a pumping laser for systems with pumped solid state lasers, the total laser radiation obtained on the light exit sides of the optical fibers serving as the pumping energy for the subsequent pumped laser.

14. Use of the assembly of any of claims 1 to 8 for tissue removal, coagulation, heat treatment, stimulation or other optical treatments in medicine or for materials processing, the laser radiation obtained on the light exit side being used to produce the stated effects.

15. Use of the assembly of claim 12 for marking an object with a pattern representing whole letters, numbers or other symbols in matrix form or representing parts of letters, numbers or other symbols so that several matching parts result side by side in whole letters, numbers or other symbols in matrix form, the ends of the combined fibers being imaged on a given place on an object (image plane) either directly, if the distance to the image plane is small relative to the total diameter of the fiber bundle, or with the aid of an optical system, so that the laser light of the luminous fiber ends produces on the surface of the object a change which causes a desired marking of the object.

16. Use of the assembly of claim 12 for flexible materials processing or tissue treatment, the ends of the combined fibers being imaged on a given place on the workpiece or tissue (image plane) either directly, if the distance to the image plane is small relative to the total diameter of the fiber bundle, or with the aid of an optical system, the desired materials processing or tissue treatment then occurring at this place.

## Revendications

1. Dispositif pour la concentration et le couplage d'émissions engendrées par un groupement de lasers à semi-conducteur, ci-après également appelé groupement de diodes laser, ou par une structure bidimensionnelle de lasers à semi-conducteur, ci-après également appelée pile de diodes laser, dans un nombre correspondant de fibres optiques individuelles, en particulier des fibres optiques multimodes, dans lequel dans la zone entre la surface radiante du groupement ou de la pile de diodes laser et les faces individuelles d'entrée de lumière des fibres optiques est prévu un système optique pour la concentration du rayonnement, dans lequel le système optique (20) est formé par une lentille cylindrique (60) dimensionnée selon le nombre et la disposition des fibres optiques individuelles (21), qui est alignée essentiellement en parallèle aux directions radiantes en multimode de chaque diode laser (23) du groupement (30), ou de la pile, dont la longueur (L_{Z}) est adaptée essentiellement à la largeur totale des fenêtres de sortie de rayon, qui définissent les surfaces radiantes, des diodes laser (23), et dont le diamètre (D_{Z}) correspond essentiellement à l'ordre de grandeur du diamètre de noyau (D_{K}) de la fibre optique (4) individuelle respective, est de préférence inférieur à ce diamètre de noyau (D_{K}), caractérisé en ce que cette lentille cylindrique (60) est collée directement sur toutes les faces d'entrée de lumière associées (24) des fibres optiques (21) s'étendant essentiellement perpendiculairement à la direction d'extension de la lentille cylindrique (60) ou est fondue dans ces faces d'entrée de lumière ou fondue sur celles-ci.

2. Dispositif selon la revendication 1, caractérisé en ce que la lentille cylindrique (60) est réalisée comme une lentille en fibre de verre.

3. Dispositif selon l'une quelconque des revendications 1 ou 2, caractérisé en ce que les fibres optiques (21) possèdent respectivement une section essentiellement ronde ou circulaire.

4. Dispositif selon l'une quelconque des revendications 1 ou 2, caractérisé en ce que les fibres optiques (21) possèdent respectivement une section essentiellement rectangulaire.

5. Dispositif selon l'une quelconque des revendications 1 à 4, caractérisé en ce qu'entre le groupement ou la pile de diodes laser (30) et la lentille cylindrique (60) du système optique, vu dans les directions radiantes, un jeu relativement petit est prévu pour la concentration du rayonnement.

6. Dispositif selon l'une quelconque des revendications 1 à 5, caractérisé en ce que pour le couplage du groupement de diodes laser (30) ou de la pile de diodes laser au nombre de fibres optiques individuelles associées à celui-ci en conjonction avec la lentille cylindrique (60), un raccord (50) commun à toutes les fibres optiques est prévu, et en ce que le groupement de diodes laser (30) ou la pile de diodes laser ainsi que le raccord (50) sont ajustés mutuellement et fixés sur un support commun (70).

7. Dispositif selon la revendication 6, caractérisé en ce que le support commun (70) est tempéré pour le réglage de la longueur d'onde laser souhaitée.

8. Dispositif selon l'une quelconque des revendications 1 à 7, caractérisé en ce que toutes les fibres optiques individuelles (21 ou 21') sont concentrées de telle sorte que les extrémités libres, c'est-à-dire les faces de sortie de lumière, des fibres optiques sont disposées selon des configurations pouvant être choisies ou prédéfinies librement.

9. Dispositif selon la revendication 8, caractérisé par une configuration telle que les extrémités libres des fibres optiques (21 ou 21') sont regroupées dans un entassement le plus dense géométriquement.

10. Dispositif selon les revendications 8 ou 9, caractérisé en ce que la configuration est choisie de telle sorte que les extrémités libres de toutes les fibres optiques (21 ou 21') forment une matrice essentiellement rectangulaire.

11. Dispositif selon les revendications 8 ou 9, caractérisé en ce que la configuration est choisie de telle sorte que les extrémités libres de toutes les fibres optiques (21 ou 21') forment une rangée.

12. Dispositif selon l'une quelconque des revendications 8 à 11, caractérisé en ce que les diodes laser individuelles (23) sont commandées respectivement de façon sélective et individuelle, de sorte que le rayonnement sort d'une sélection correspondante de faces de sortie de lumière des fibres optiques (21 ou 21'), ce qui permet de générer des dessins de sortie de rayon pouvant être choisis ou prédéfinis librement.

13. Utilisation d'un dispositif selon l'une quelconque des revendications 1 à 8 en tant que laser de pompage pour des systèmes avec des lasers solides pompés, dans laquelle le rayonnement laser obtenu au total au niveau des faces de sortie de lumière des fibres optiques sert d'énergie de pompage pour le laser pompé disposé en aval.

14. Utilisation d'un dispositif selon l'une quelconque des revendications 1 à 8 pour l'abrasion de tissus, la coagulation, le traitement thermique, la stimulation ou d'autres traitements optiques en médicine ou pour le traitement de matériau, dans laquelle le rayonnement laser obtenu au niveau de la face de sortie de lumière est utilisé pour la génération des effets mentionnés.

15. Utilisation d'un dispositif selon la revendication 12 pour marquer un objet avec un dessin, qui représente des lettres, des chiffres ou d'autres symboles entiers sous forme de matrice ou qui représente des parties de lettres, de chiffres ou d'autres symboles de telle sorte que plusieurs parties allant ensemble produisent des lettres, des chiffres ou d'autres symboles entiers sous forme de matrice quand elles sont juxtaposées, dans laquelle les extrémités de fibre des fibres regroupées sont reproduites à un endroit prédéfini d'un objet (plan image) soit directement, quand la distance par rapport au plan d'image est relativement faible en relation avec le diamètre total du faisceau de fibres, soit à l'aide d'un système optique, de sorte qu'à la surface de l'objet, une modification provoquant un marquage souhaité de l'objet est obtenue par la lumière laser des extrémités laser lumineuses.

16. Utilisation d'un dispositif selon la revendication 12 pour un traitement de matériau ou un traitement de tissu souple, dans laquelle les extrémités de fibre des fibres regroupées sont reproduites soit directement, quand la distance au plan d'image est relativement faible par rapport au diamètre total du faisceau de fibres, soit à l'aide d'un système optique, à un endroit prédéfini de la pièce à usiner ou du tissu (plan image) sur lequel a lieu ensuite le traitement de matériau ou le traitement de tissu.
